# EUROPEAN PATENT APPLICATION

(11) **EP 4 344 721 A1**
(43) Date of publication of application: **03.04.2024**
(21) Application number: 22895259.4
(22) Date of filing: 03.10.2022
(51) Int. Cl.: A61M 25/00

(54) **CATHETER**

(30) Priority: 16.11.2021 JP 2021186242
(71) Applicant: Goodman Co., Ltd., Aichi 460-0008 (JP)
(72) Inventor: CHIZUKI, Toshihiko, Seto-shi, Aichi 489-0976 (JP)
(74) Representative: Prüfer & Partner mbB Patentanwälte · Rechtsanwälte
(86) International application number: PCT/JP2022/037002
(87) International publication number: WO 2023/089973

(57) **Abstract**

A catheter 10 includes a catheter tube 11 and a connector 12 connected to a base-end portion 11a of the catheter tube 11. The connector 12 includes a body 17 and an extending portion 18. The body 17 includes an insertion hole 16 in which the base-end portion 11a of the catheter tube 11 is inserted. The extending portion 18 extends from the body 17 on the tip-end side and has a tubular shape. The extending portion 18 is away from a non-inserted tube portion 22 of the catheter tube 11 out of the insertion hole 16 on the tip-end side to surround a part of the non-inserted tube portion 22.

## Description

### Cross-Reference to Related Application

The present application is based on Japanese Patent Application No. 2021-186242 filed on November 16, 2021, the entire contents of which are incorporated herein by reference.

### Technical Field

The present disclosure relates to a catheter.

### Background Art

In general, a catheter includes a catheter tube that is inserted into the body and a connector that is connected to a base-end portion of the catheter tube (e.g., PLT1) . The connector includes an insertion hole in which the base-end portion of the catheter tube is inserted. The base-end portion is inserted in the insertion hole and joined to the connector by welding or bonding.

### Citation List

### Patent Literature

PLT1: Japanese Unexamined Patent Application Publication No. 2013-090717.

### Summary of Invention

### Technical Problem

During insertion of the catheter into the body, a non-inserted tube portion of the catheter tube out of the insertion hole on a tip-end side of the catheter may be curved relative to a joint section that is joined to the connector. If the non-inserted tube portion is significantly curved, the catheter tube may bend at a border between a joint section that is joined to the connector and the non-inserted tube portion. This may result in kinks. In particular, a difference in hardness between the joint section that is joined to the connector and the non-inserted tube portion of the catheter tube is large and thus the kinks may easily occur at the border.

The present disclosure has been made in view of the above circumstances. A main purpose of the present disclosure is to provide a catheter in which kinks are less likely to occur.

### Solution to Problem

To solve the problem described above, a first aspect of the present disclosure provides a catheter that includes a catheter tube and a connector that is connected to a base-end portion of the catheter tube. The connector includes an insertion hole in which the base-end portion of the catheter tube is inserted. The connector is joined to the base-end portion of the catheter tube in the insertion hole. The connector includes a surrounding portion having a tubular shape or an annular shape. The surrounding portion is located closer to a tip-end side than the insertion hole and away from a non-inserted tube portion of the catheter tube to surround a part of the non-inserted tube portion at a distance. The non-inserted tube portion is out of the insertion hole on a tip-end side.

According to the first aspect, the base-end portion of the catheter tube is inserted in the insertion hole of the connector and joined to the connector. The part of the non-inserted tube portion of the catheter tube out of the insertion hole on the tip-end side is surrounded by the surrounding portion of the connector. According to the configuration, when the non-inserted tube portion is curved during the insertion of the catheter into the body, the surrounding portion restricts significant curving of the non-inserted tube portion. Therefore, the kinks are less likely to occur at the border between the joint section that is joined to the connector and the non-inserted tube portion.

In a second aspect of the present disclosure, the connector of the catheter in the first aspect includes a body that includes the insertion hole and an extending portion that extends from the body on the tip-end side and has a tubular shape. The extending portion includes the surrounding portion.

While the significant curving of the non-inserted tube portion of the catheter tube is restricted by the surrounding portion, a load may be applied to the surrounding portion by the non-inserted tube portion outward in the radial direction. According to the second aspect, the connector includes the body that includes the insertion hole and the extending portion that extends from the body on the tip-end side and has the tubular shape, and the extending portion is the surrounding portion that surrounds the part of the non-inserted tube portion. According to the configuration, the extending portion (the surrounding portion) can stably receive the load applied to the extending portion.

In a third aspect of the present disclosure, the extending portion of the catheter in the second aspect has an inner surface that includes a tapered surface. The tapered surface has a diameter that increases toward the tip-end side.

According to the third aspect, when the non-inserted tube portion of the catheter tube is curved, the non-inserted tube portion may curves along tapered surface of the extending portion and the contact the tapered surface of the extending portion. According to the configuration, a stress is less likely to concentrate on the non-inserted tube portion. Therefore, the kinks due to the concentration of stress are less likely to occur.

In a fourth aspect of the present disclosure, the tapered surface of the catheter in the third aspect joins an inner wall of the insertion hole.

According to the fourth aspect, the tapered surface joins the inner wall of the insertion hole and thus no step is present at a border between the tapered surface and the inner wall of the insertion hole. According to the configuration, the bending of the catheter tube is further properly restricted at the border between the joint section that is inserted in the insertion hole and joined to the connector and the non-inserted tube portion that is out of the insertion hole. Therefore, the kinks are further less likely to occur at the border.

In a fifth aspect of the present disclosure, an inner diameter of the extending portion of the catheter at a base-end section of the extending portion in the second aspect is greater than a diameter of the insertion hole. The inner diameter of the extending portion is constant from the base-end section to a tip-end section of the extending portion, or the inner diameter of the extending portion increases from the base-end section toward the tip-end section of the extending portion.

If a force is applied to the catheter tube in a direction toward the base-end side during the insertion of the catheter into the body, a section of the non-inserted tube portion of the catheter tube adjacent to the base-end portion of the catheter tube may curve such that the section protrudes (or bulges) in a direction perpendicular to the axial direction. According to the fifth aspect, the extending portion has the configuration described above and thus a large size of an internal space may be provided in the extending portion from the base-end section to the tip-end section. Therefore, a base-end section of the non-inserted tube portion can be curved without stress in the internal space of the extending portion and thus kinks are less likely to occur around the base-end section of the non-inserted tube portion.

In a sixth aspect of the present disclosure, an inner surface of a tip-end section of the surrounding portion of the catheter in any one of the first to the fifth aspects is curved such that an inner diameter of the surrounding portion increases toward a distal end of the surrounding portion.

If a force to curve the non-inserted tube portion is applied to the non-inserted tube portion while significant curving of the non-inserted tube portion is restricted by the extending portion, the non-inserted tube portion may bend at the tip-end section of the extending portion. According to the sixth embodiment, the inner surface of the extending portion includes the curved surface on the tip-end section and thus the non-inserted tube portion may be curved along the curved surface at the tip-end section of the extending portion. According to the configuration, the non-inserted tube portion is less likely to be bent at the tip-end section of the extending portion. Therefore, kinks due to the bending of the non-inserted tube portion are less likely to occur.

In a seventh aspect of the present disclosure, the base-end portion of the catheter tube inserted in the insertion hole of the catheter in any one of the first to the sixth aspects includes a joint section that is joined to the connector and a non-joint section that may be located closer to the tip-end side than the joint section and not joined to the connector.

According to the seventh aspect, the base-end portion of the catheter tube inserted in the through hole includes the joint section that is joined to the connector and the non-joint section that is located closer the tip-end side than the joint section and not joined to the connector. According to the configuration, a difference (or a variation) in hardness between the joint section that is joined to the connector and the non-inserted tube portion that is out of the insertion hole is reduced in the catheter tube. Therefore, kinks due to the difference in hardness are less likely to occur.

In an eighth aspect of the present disclosure, the connector of the catheter in any of the first to the seventh aspects includes a body that includes the insertion hole. The body and the surrounding portion are in a single piece.

As described earlier, while significant curving of the non-inserted tube portion of the catheter tube is restricted by the surrounding portion, a load may be applied to the surrounding portion by the non-inserted tube portion outward in the radial direction. According to the eighth aspect, the body that has the insertion hole and the surrounding portion of the connector are in the single piece and thus separation of the surrounding portion from the body due to the load is less likely to occur.

In a ninth aspect of the present disclosure, the connector of the catheter in any one of the first to the eighth aspects has a light transmitting property and the catheter tube has a light absorbing property. The connector is welded to the base-end portion of the catheter tube.

According to the ninth aspect, the connector has the light transmitting property and the catheter tube has the light absorbing property. The connector and the catheter tube can be laser-welded at an interface between the connector and the catheter tube by applying laser light to the interface between the connector and the catheter tube through the connector. According to the configuration, a thickness of a welded section between the connector and the catheter tube can be relatively small and thus the difference in hardness between the joint section (or a welded section) of the catheter tube welded to the connector and the non-inserted tube portion can be reduced. Therefore, the kinks due to the difference in hardness are further less likely to occur.

### Brief Description of Drawings

The above-described object and any other object, features, and advantages of the present disclosure will be further clarified by the following detailed description made with reference to the attached drawings.
[Figure 1] A cross-sectional view illustrating a portion of a catheter including a joint section between a catheter tube and a connector in a first embodiment.
[Figure 2] A cross-sectional view for describing functions of an extending portion.
[Figure 3] A cross-sectional view illustrating a portion of a catheter including a joint section between a catheter tube and a connector in a second embodiment.
[Figure 4] A cross-sectional view for describing functions of an extending portion.
[Figure 5] A cross-sectional view of an extending portion of a connector and therearound in another embodiment.
[Figure 6] A cross-sectional view of an extending portion of a connector and therearound in another embodiment.
[Figure 7] A cross-sectional view of an extending portion of a connector and therearound in another embodiment.

### Description of Embodiments

### [First Embodiment]

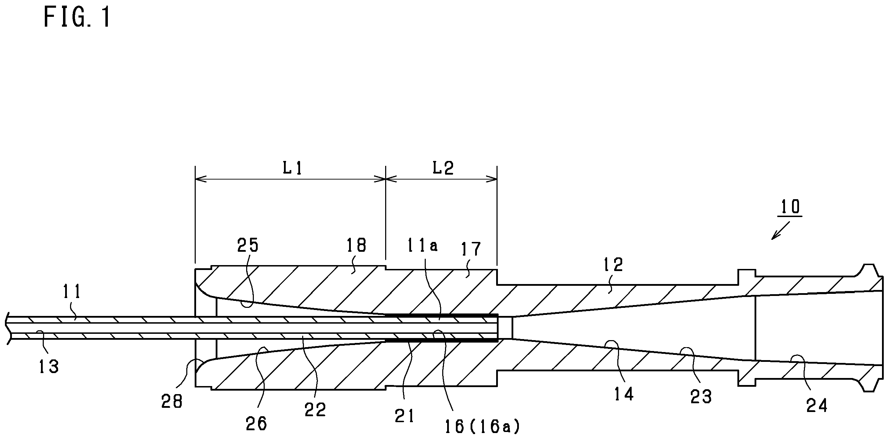

An embodiment of the present disclosure will be described with reference to the drawings. FIG. 1 is a cross-sectional view illustrating a portion of a catheter 10 including a joint section between the catheter tube 11 and a connector 12.

As illustrated in FIG. 1, the catheter 10 includes the catheter tube 11 and the connector 12 that is connected to a base-end portion (a proximal end portion) of the catheter tube 11. The catheter tube 11 has a round tubular shape and includes an internal lumen 13 that extends in an axial direction for an entire length of the catheter tube 11. The catheter tube 11 is made of a soft resin such as polyamide to have flexibility. The catheter tube 11 is in black to have light absorbing properties.

Note that the catheter 10 may be, but not limited to, a contrast catheter. The catheter 10 may be another type of catheter such as a balloon catheter and a suction catheter.

The connector 12 is made of a hard resin such as polyamide. The connector 12 has a round tubular shape and a hole 14 that passes through the connector 12 in an axial direction of the connector 12. The connector 12 is transparent or semitransparent, that is, the connector 12 has light transmitting properties. The connector 12 may be referred to as a hub. The connector 12 may be made of another type of hard resin such as polyacetal, polycarbonate, and ABS.

The connector 12 includes a body 17 and an extending portion 18. The body 17 has an insertion hole 16 in which a base-end portion 11a of the catheter tube 11 is inserted. The extending portion 18 extends from the body 17 toward a tip-end of the catheter 10. The insertion hole 16 is a section of the hole 14 and has a diameter that is about equal to an outer diameter of the catheter tube 11. The diameter of the insertion hole 16 is constant for an entire length of the body 17 in the axial direction.

The base-end portion 11a of the catheter tube 11 inserted in the insertion hole 16 is joined to the body 17 by welding. In this embodiment, the base-end portion 11a of the catheter 11 is joined to the body 17 by laser welding. The body 17 and the base-end portion 11a of the catheter tube 11 are laser welded at an interface between the body 17 and the base-end portion 11a. In the laser welding, laser light is applied to the interface between the body 17 and the base-end portion 11a of the catheter tube 11 through the connector 12. With the laser light, heat is generated at the interface, the body 17 and the base-end portion 11a melts due to the heat, and the body 17 and the base-end portion 11a are welded together.

The body 17 extends more toward a base-end side of the catheter 10 than the insertion hole 16 and includes a base-end portion of the connector 12. The body 17 includes a tapered hole 23 and a connection hole 24. The tapered hole 23 extends from the insertion hole 16 on the base-end side. The connection hole 24 extends from the tapered hole 23 on the base-end side. The tapered hole 23, the connection hole 24, and the insertion hole 16 are sections of the hole 14. The tapered hole 23 has a diameter that increased toward the base-end. The connection hole 24 includes an opening at the base-end portion of the connector 12. The connection hole 24 can receive another connector for connection.

Next, the extending portion 18 will be described. The extending portion 18 extends from the tip-end section of the body 17 on the tip-end side and has a cylindrical shape. An extending length L1 of the extending portion 18 is greater than a length L2 (a length in the axial direction) of the joint section 21 of the catheter tube 11 joined to the body 17.

The outer diameter of the extending portion 18 is about constant for the entire length in the axial direction but the inner diameter of the extending portion 18 continuously increases from a base-end side of the catheter 10 toward the tip-end side. The inner diameter of the extending portion 18 is greater than the diameter of the insertion hole 16, that is, greater than the outer diameter of the catheter tube 11. The inner diameter of the tip-end section of the extending portion 18 is greater than twice the outer diameter of the catheter tube 11. Because the inner diameter of the extending portion 18 increases toward the tip-end side, the extending portion 18 may be referred to as a diameter increasing portion.

The inner surface of the extending portion 18 includes a tapered surface 26, the diameter of which increases from the based end toward the tip-end side. The tapered surface 26 increases in diameter from an inner wall 16a of the insertion hole 16 toward the tip-end side. The tapered surface 26 joins the inner wall 16a of the insertion hole 16 at a border between the tapered surface 26 and the inner wall 16.

The extending portion 18 has an internal space 25. The internal space 25 communicates with the insertion hole 16. The internal space 25 is a section of the hole 14. The internal space 25 opens toward the tip-end side. The catheter tube 11 includes a non-inserted tube portion 22 that is out of the insertion hole 16 on the tip-end side and extends through the internal space 25. A part of the non-inserted tube portion 22 is surrounded by the extending portion 18. The non-inserted tube portion 22 and the extending portion 18 are spaced apart from each other. Note that the extending portion 18 may correspond to a "surrounding portion."

An inner surface of a tip-end section of the extending portion 18 is curved such that an inner diameter of the extending portion 18 increases toward the tip-end of the extending portion 18 and defined as a curved surface 28. The curved surface 28 smoothly connects a tip-end surface of the extending portion 18 to the tapered surface 26. The radius of curvature of the curved surface 28 is greater than the outer radius of the catheter tube 11. Specifically, the radius of curvature of the curved surface 28 is greater than the outer radius of the catheter tube 11 for the entire curved surface portion 28. Note that the radius of curvature of a portion of the curved surface 28 may be less than or equal to the outer radius of the catheter tube 11.

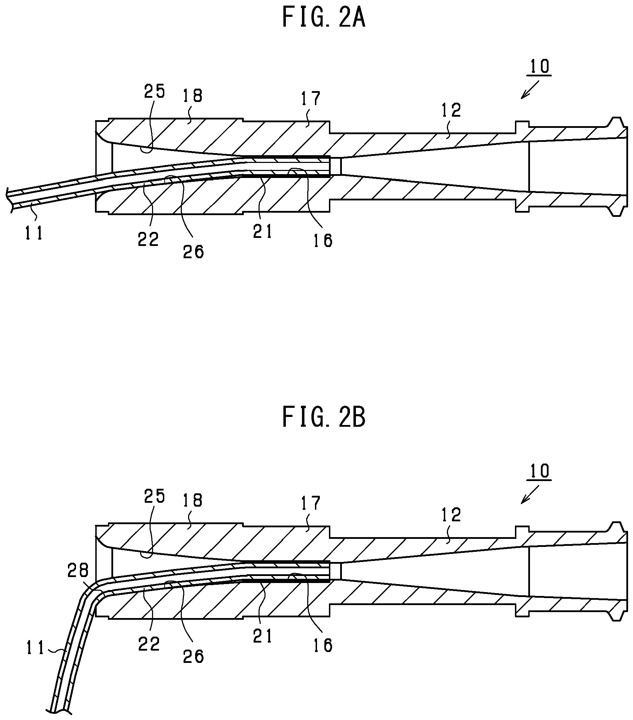

Functions of the extending portion 18 will be described with reference to FIG. 2. FIG. 2 is a cross-sectional view for describing the functions of the extending portion 18.

During insertion of the catheter 10 into a patient's body, the non-inserted tube portion 22 of the catheter tube 11 out of the insertion hole 16 may be curved relative to the joint section 21 of the catheter tube 11 joined to the connector 12. In such a situation, according to the connector 12 described above, the non-inserted tube portion 22 contacts the extending portion 18 of the connector 12, more specifically, curves along the tapered surface 26 of the extending portion 18 and contacts the tapered surface 26 of the extending portion. Due to the contact, significant curving of the non-inserted tube portion 22 relative to the joint section 21 is restricted. Therefore, kinks are less likely to occur at the border between the non-inserted tube portion 22 and the joint section 21 of the catheter tube 11.

If the non-inserted tube portion 22 is further curved from the state in which the significant curving of the non-inserted tube portion 22 is restricted by the extending portion 18, the non-inserted tube portion 21 may bend at the tip-end section of the extending portion 18. According to the configuration of the connector 12, the non-inserted tube portion 22 curves along the curved surface 28 as illustrated in FIG. 2B. The non-inserted tube portion 22 is less likely to be bent at the distal edge of the extending portion 18. Therefore, kinks due to bending are less likely to occur.

According to the configuration of the embodiment described above, the following advantageous effects can be achieved.

While the significant curving of the non-inserted portion 22 of the catheter tube 11 is restricted by the surrounding portion, a load may be applied to the surrounding portion by the non-inserted tube portion 22 outward in the radial direction. In the embodiment, the extending portion 18 that has the tubular shape and extends from the body 17 of the connector 12 on the tip-end side corresponds to the surrounding portion and thus the extending portion 18 (the surrounding portion) can stably receive the load applied to the extending portion 18.

Because the body 17 and the extending portion 18 are in a single piece, separation of the extending portion 18 from the body 17 due to the load is less likely to occur.

Because the inner surface of the extending portion 18 includes the tapered surface 26 with the diameter that increases toward the tip-end side, the non-inserted tube portion 22 contacts along the tapered surface 26 when the non-inserted tube portion 22 is curved. Therefore, a stress is less likely to concentrate on the non-inserted tube portion 22 and thus kinks due to the concentration of stress are less likely to occur.

Because the tapered surface 26 and the inner wall 16a of the insertion hole 16 join together, no step is present at the border between the tapered surface 26 and the inner wall 16a of the insertion hole 16. According to the configuration, the catheter tube 11 is less likely to be bent at the border between the joint section 21 joined to the connector 12 in the insertion hole 16 and the non-inserted tube portion 22 out of the insertion hole 16. Therefore, the kinks are further less likely to occur at the border.

The connector 12 has the light transmitting properties and the catheter tube 11 has the light absorbing properties. By applying laser light to the interface between the connector 12 and the catheter tube 11 through the connector 12, the connector 12 and the catheter tube 11 are laser welded at the interface. According to the configuration, a thickness of the welded section between the connector 12 and the catheter tube 11 is relatively small and thus a difference in hardness between the joint section 21 joined to the connector 12 (the welded section) and the non-inserted tube portion 22 of the catheter tube 11 is small. Therefore, the kinks due to the difference in hardness are further less likely to occur.

### [Second Embodiment]

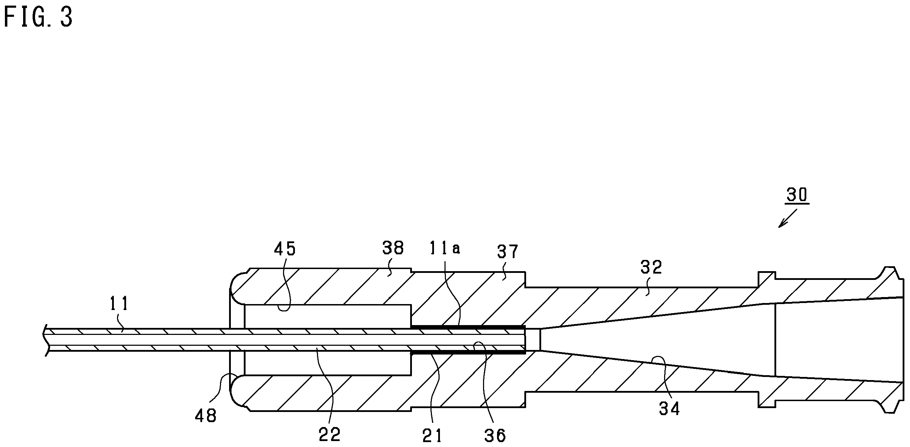

A second embodiment will be described. The second embodiment includes a connector that includes an extending portion, the configuration of which is different from that of the first embodiment. The configuration of the second embodiment will be described with a focus on differences between the first embodiment and the second embodiment. FIG. 3 is a cross-sectional view illustrating a portion of a catheter 30 including a joint section between the catheter tube 11 and a connector 32 in a second embodiment.

As illustrated in FIG. 3, the catheter 30 in this embodiment includes the catheter tube 11 and the connector 32 that is coupled to the base-end portion 11a of the catheter tube 11, similar to the first embodiment. Components and sections of the second embodiment the same as those in the first embodiment are identified with the same reference signs and will not be described.

Similar to the first embodiment, the connector 32 includes a body 37 and an extending portion 38. The body 37 includes an insertion hole 36 in which the base-end portion 11a of the catheter tube 11 is inserted. The extending portion 38 extends from the body 37 on the tip-end side and has a cylindrical shape. The configuration of the body 37 is similar to that of the first embodiment and thus will not be described.

The extending portion 38 has an inner diameter that is constant from the base-end section to the tip-end section. Therefore, the inner surface of the extending portion 38 extends parallel to the axial direction, that is, the inner surface does not include a tapered surface. The inner diameter of the extending portion 38 is greater than the diameter of the insertion hole 16, that is, greater than the outer diameter of the catheter tube 11. Specifically, the inner diameter of the extending portion 38 is greater than twice the outer diameter of the catheter tube 11. Because the inner diameter of the extending portion 38 is constant from the base-end section to the tip-end section, the extending portion 38 may be referred to as a constant diameter section.

Further, an extending length of the extending portion 38 is greater than a length of the joint section 21 of catheter tube 11 joined to the connector 12, similar to the first embodiment. Further, an inner surface of a tip-end section of the extending portion 38 is curved and defined as a curved surface 48.

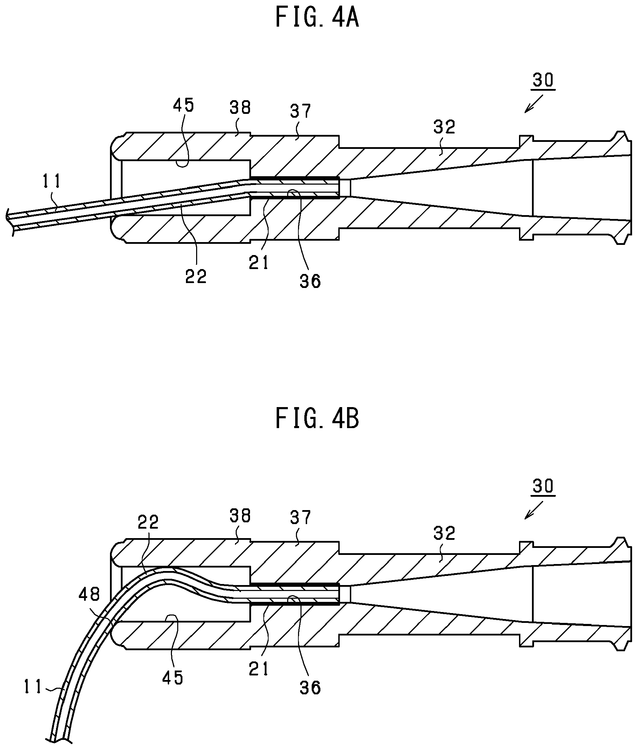

Next, functions of the extending portion 38 will be described with reference to FIG. 4. FIG. 4 is a cross-sectional view for describing functions of the extending portion 38.

As illustrated in FIG. 4A, when the non-inserted tube portion 22 of the catheter tube 11 is curved relative to the joint section 21 of the catheter tube 11 during insertion of the catheter 30 into the body, the non-inserted tube portion 22 contacts the extending portion 38 of the connector 32. Due to the contact, the significant curving of the catheter tube 11 is restricted. Therefore, kinks are less likely to occur at the border between the non-inserted tube portion 22 and the joint section 21 of the catheter tube 11, similar to the first embodiment.

During the insertion of the catheter 30 into the body, a force may be applied to the catheter tube 11 in a direction toward the base-end. In such a situation, the non-inserted tube portion 22 around the base-end section of the non-inserted tube portion 22 may curve in a direction perpendicular to the axial direction to be convex to (or to bulge in the direction perpendicular to) the axial direction. Because the inner diameter of the extending portion 38 of the connector 32 is greater than the diameter of the insertion hole 16 for the entire length of the extending portion 38 from the base-end section to the tip-end section, a large size of an internal space 45 is provided in the extending portion from the base-end section to the tip-end section. Therefore, as illustrated in FIG. 4B, the base-end section of the non-inserted tube portion 22 can be curved without stress in the internal space 45 of the extending portion 38 and thus the kinks are less likely to occur around the base-end section of the non-inserted tube portion 22.

### [Other Embodiments]

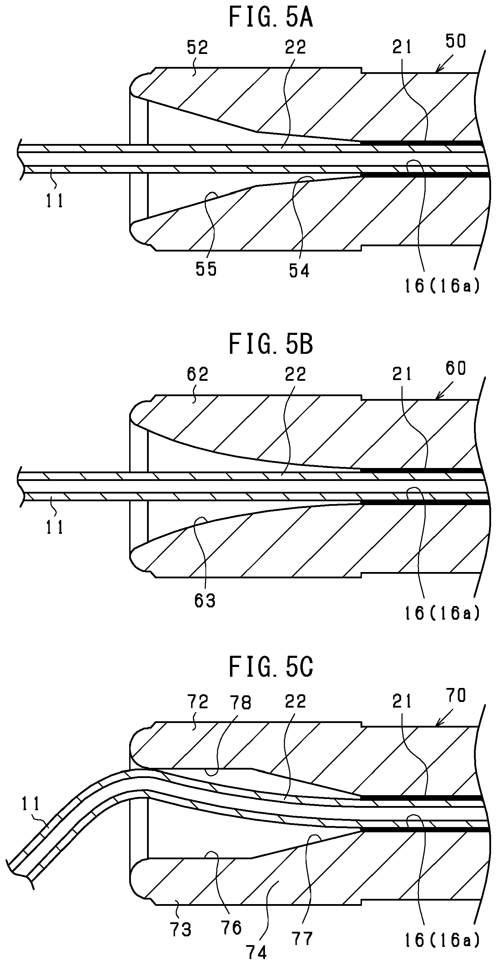

The present disclosure is not limited to the above embodiments and may be implemented as the followings, for example.
(1) A connector 50 illustrated in FIG. 5A may include an extending portion 52, an inner surface of which may include tapered surfaces 54 and 55. The tapered surfaces 54 and 55 are adjacent to each other in the axial direction of the extending portion 52. The tapered surface 54 is located on the base-end side and the tapered surface 55 is located on the tip-end side. The tapered surfaces 54 and 55 increase in diameter toward the tip-end side and join together at a border between the tapered surfaces 54 and 55. A base-end section of the tapered surface 54 on the base-end side joins the inner wall 16a of the insertion hole 16. Slope angles of the tapered surfaces 54 and 55 relative to the axial direction of the extending portion 52 are different from each other. Specifically, the slope angle of the tapered surface 55 on the tip-end side is greater than the slope angle of the tapered surface 54 on the base-end side.
   According to the configuration, the slope angle of the tapered surface 54 on the base-end side is smaller and thus the non-inserted tube portion 22 of the catheter tube 11 is further less likely to be bent relative to the joint section 21. Therefore, the kinks are further less likely to occur. Further, the slope angle of the tapered surface 55 on the tip-end side is larger and thus a sufficient space is provided to allow the non-inserted tube portion 22 to curve without kinks.
(2) A connector 60 illustrated in FIG. 5B may include an extending portion 62. The extending portion 62 may include an inner surface that may include a curved surface 63. The curved surface 63 of the embodiment illustrated in FIG. 5B extends for the entire length of the extending portion 62 in the axial direction. The curved surface 63 has an inner diameter that increases from the base-end side toward the tip-end side. The curvature of the curved surface 63 increases from the base-end toward the tip-end side. According to the configuration, the same effects as the configuration described in (1) above can be achieved.
(3) An connector 70 illustrated in FIG. 5C may include an extending portion 72 that includes a constant diameter section 73 and an increasing diameter section 74. In the embodiment illustrated in FIG. 5C, the constant diameter section 73 is located on the tip-end side and the increasing diameter section 74 is located on the base-end side. The constant diameter section 73 has an inner diameter that is constant. Therefore, the inner surface of the constant diameter section 73 extends parallel to the axial direction of the extending portion 72 and thus the inner surface is defined as a non-tapered surface 76. The inner diameter of the constant diameter section 73 is greater than the diameter of the insertion hole 16.

The increasing diameter section 74 has an inner diameter that increases from the base-end side toward the tip-end side. Therefore, the inner surface of the increasing diameter section 74 has a diameter that increases toward the tip-end side and thus the inner surface is defined as a tapered surface 77. The base-end section of the tapered surface 77 joins the inner wall 16a of the insertion hole 16 and the tip-end section of the tapered surface 77 joins the non-tapered surface 76. The lengths of the constant diameter section 73 and the increasing diameter section 74 in the axial direction are about equal to each other.

According to the configuration, if a force is applied to the catheter tube 11 in a direction toward the base-end side, the non-inserted tube portion 22 can be curved in an internal space 78 of the extending portion 72 (mainly inside the constant diameter section 73). Therefore, in such a situation, the kinks are less likely to occur.

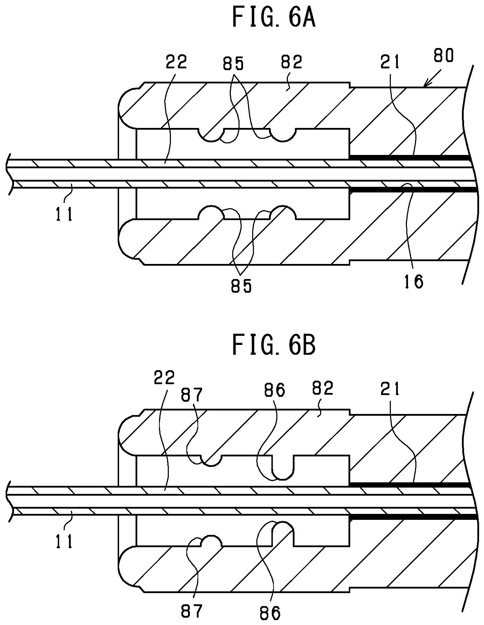

(4) A connector 80 illustrated in FIG. 6A may include an extending portion 82 that includes protrusions 85 protruding inward. Each of the protrusions 85 extends in a circumferential direction of the extending portion 82 and has an annular shape. The protrusions 85 (specifically, two protrusions) are at predefined intervals in the axial direction of the extending portion 82. According to the configuration, when the non-inserted tube portion 22 of the catheter tube 11 is curved, the non-inserted tube portion 22 contact not only the tip-end section of the extending portion 82 but also the tops of the protrusions 85. Therefore, the stress that may be applied to the non-inserted tube portion 22 at points of contact with the connector 80 can be dispersed.

In the embodiment illustrated in FIG. 6A, the heights of the protrusions 85 may be all equal. However, protrusions 86 and 87 that have different heights may be provided as illustrated in FIG. 6B. In the embodiment illustrated in FIG. 6B, the height of the protrusion 87 on the tip-end side is less than the height of the protrusion 86 on the base-end side. Namely, in the embodiment illustrated in FIG. 6B, the heights of the protrusions on the tip-end side are smaller. According to the configuration, when the non-inserted tube portion 22 is curved, the non-inserted tube portion 22 can easily contact the tops of the protrusions 86 and 87. Therefore, the stress that may be applied to the non-inserted tube portion 22 at points of contact with the connector 80 is effectively dispersed.

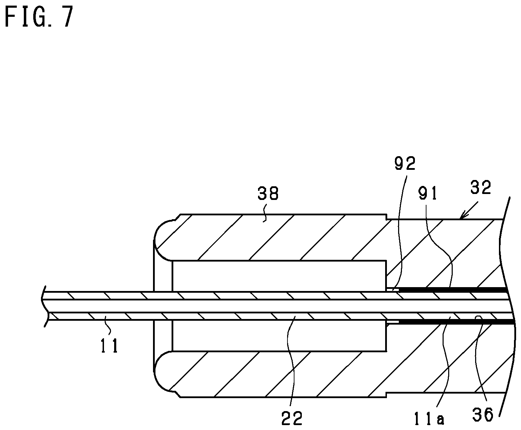

(5) As illustrated in FIG. 7, the base-end portion 11a of the catheter tube 11 inserted in the insertion hole 36 of the connector 32 may include a joint section 91 that is joined to the connector 32 and a non-joint section that is located closer to the tip-end side than the joint section 91 and not joined to the connector 32. According to the configuration, a deference in hardness (or a variation in hardness) between the joint section 91 that is joined to the connector 32 and the non-inserted tube portion that is out of the insertion hole 36 can be reduced. Therefore, kinks due to the difference in hardness are less likely to occur.

(6) In each of the above embodiments, the connector 12, 32 may include the body 17, 37 and the extending portion 18, 38; however, the configuration of the connector is not limited to such a configuration. For example, a connector may include a body, a surrounding portion, and a connecting portion. The surrounding portion is located closer to the tip-end side than the body to surround a part of the non-inserted tube portion 22. The connecting portion has a rod shape and connect the surrounding portion to the body. According to the configuration, when the non-inserted tube portion 22 of the catheter tube 11 is curved, the surrounding portion restricts significant curving of the non-inserted tube portion 22. Therefore, kinks are less likely to occur at the border between the joint section 21 and the non-inserted tube portion 22 of the catheter tube 11.

(7) In each of the above embodiments, the base-end portion 11a of the catheter tube 11 is laser welded to the connector 12; however, welding other than the laser welding may be used. The base-end portion 11a of the catheter tube 11 may be bonded to the connector 12 with an adhesive. Namely, the base-end portion 11a of the catheter tube 11 may be joined to the connector 12 by welding or with an adhesive.

The present disclosure has been described with reference to the embodiments. However, the present disclosure is not limited to the embodiments or the structures. Various modifications and modifications within a scope of equivalents may be within in the technical scope of the present disclosure. Further, various combinations, configurations, and other combinations and configurations including single elements, more or less, may be within the technical scope of the present disclosure.

### Reference Sings List

10: Catheter, 11: Catheter tube, 12: Connector, 16: Insertion hole, 17: Body, 18: Extending portion as enclosure portion, 22: Non-inserted tube portion, 26: Tapered surface, 28: Curved surface, 30: Catheter, 32: Connector, 36: Insertion hole, 37: Body, 38: Extending portion

## Claims

1. A catheter comprising:
a catheter tube; and
a connector connected to a base-end portion of the catheter tube, wherein
the connector includes an insertion hole in which the base-end portion of the catheter tube is inserted,
the connector is joined to the base-end portion of the catheter tube in the insertion hole,
the connector includes a surrounding portion having a tubular shape or an annular shape,
the surrounding portion is located closer to a tip-end side than the insertion hole and away from a non-inserted tube portion of the catheter tube to surround a part of the non-inserted tube portion at a distance, and
the non-inserted tube portion is out of the insertion hole on a tip-end side.

2. The catheter according to claim 1, wherein
the connector includes a body that includes the insertion hole and an extending portion that extends from the body on the tip-end side and has a tubular shape, and
the extending portion is the surrounding portion.

3. The catheter according to claim 2, wherein
the extending portion has an inner surface that includes a tapered surface, and
the tapered surface has a diameter that increases toward the tip-end side.

4. The balloon catheter according to claim 3, wherein the tapered surface joins an inner wall of the insertion hole.

5. The balloon catheter according to claim 2, wherein
an inner diameter of the extending portion at a base-end section of the extending portion is greater than a diameter of the insertion hole, and
the inner diameter of the extending portion is constant from the base-end section to a tip-end section of the extending portion, or the inner diameter of the extending portion increases from the base-end section toward the tip-end section of the extending portion.

6. The balloon catheter according to any one of claims 1 to 5, wherein an inner surface of a tip-end section of the surrounding portion is curved such that an inner diameter of the surrounding portion increases toward a tip-end of the surrounding portion.

7. The balloon catheter according to any one of claims 1 to 6, wherein the base-end portion of the catheter tube inserted in the insertion hole includes a joint section that is joined to the connector and a non-joint section that is located on the tip-end side than the joint section and not joined to the connector.

8. The balloon catheter according to any one of claims 1 to 7, wherein
the connector includes a body that includes the insertion hole, and
the body and the surrounding portion are in a single piece.

9. The balloon catheter according to any one of claims 1 to 8, wherein
the connector has a light transmitting property,
the catheter tube has a light absorbing property, and
the connector is welded to the base-end portion of the catheter tube.
